## Europäisches Patentamt
## European Patent Office
## Office européen des brevets

(19)

(11) Numéro de publication: **0 207 438**

**B1**

(12) # FASCICULE DE BREVET EUROPÉEN

(45) Date de publication du fascicule du brevet:
**23.08.89**

(51) Int. Cl.⁴: **A 61 N 1/05**

(21) Numéro de dépôt: **86108681.7**

(22) Date de dépôt: **25.06.86**

(54) **Stylet pour électrode implantable dans le corps.**

(30) Priorité: **27.06.85 FR 8509986**

(43) Date de publication de la demande:
**07.01.87 Bulletin 87/2**

(45) Mention de la délivrance du brevet:
**23.08.89 Bulletin 89/34**

(84) Etats contractants désignés:
**CH DE GB LI NL SE**

(56) Documents cité:
**CH-A-484 538**
**CH-A-4 169 479**
**GB-A-2 064 963**
**US-A-1 730 993**
**US-A-2 118 631**
**US-A-2 980 398**
**US-A-3 999 551**

(73) Titulaire: **Nivarox- FAR S.A., Avenue du Collège 10,
CH- 2400 Le Locle (CH)**

(72) Inventeur: **Germain, Maillard, Crêtets 10, CH- 2300
La Chaux- de- Fonds (CH)**

(74) Mandataire: **Caron, Gérard, ICB Ingénieurs
Conseils en Brevets SA Passage Max. Meuron
6, CH- 2001 Neuchâtel (CH)**

## Description

La présente invention est relative aux électrodes implantables dans le corps et elle concerne plus particulièrement les stylets qui sont associés à ces électrodes pour les rigidifier lors de leur implantation.

A l'heure actuelle, les électrodes implantables sont largement répandues et utilisées en médecine pour établir des diagnostics, soigner des malades et surtout pour équiper les patients qui ont une affection cardiaque à laquelle on tente de remédier par l'implantation à demeure d'un stimulateur.

Ces électrodes sont formées par un conducteur électrique creux extrêmement souple et gainé sur toute sa longueur d'un matériau isolant. Le conducteur lui-même est formé d'un enroulement en hélice de plusieurs torons de fil, eux-mêmes généralement formés d'hélices à leur tour. Un tel agencement permet d'assurer la souplesse de l'électrode requise pour qu'elle puisse s'adapter à chaque instant aux modifications de forme des vaisseaux sanguins, en particulier à proximité du coeur. Cependant, lors de l'implantation, pour pouvoir suivre tous les méandres des vaisseaux, il convient que l'électrode soit assez rigide et c'est précisément le stylet qui assure temporairement cette rigidité. Ainsi, ce stylet est formé par un fil en métal élastique introduit dans l'électrode et présentant à peu près la même longueur que celle-ci.

Par conséquent, lorsque le chirurgien a implanté l'électrode munie de son stylet, il retire immédiatement celui-ci et procède ensuite à la connexion de l'électrode avec le stimulateur ou un autre appareil analogue.

On sait, par ailleurs, que les électrodes, surtout celles destinées à la stimulation cardiaque, doivent s'accrocher aux tissus à exciter ce qui est réalisé en général par des configurations spéciales de la tête de l'électrode (relief particulier de la surface de la tête, barbillons inclinés sur l'axe etc.). Malgré cela, il arrive encore fréquemment que l'électrode se décroche, surtout au cours des premières semaines suivant l'implantation, car alors le tissu n'a pas encore crû suffisamment pour assurer le maintien de l'électrode. Les chirurgiens ont donc l'habitude de contrôler l'accrochage de l'électrode quelque temps après leur intervention. Cette opération peut être effectuée à l'aide d'un stylet analogue à celui qui est utilisé pour l'implantation.

Une électrode implantée suit normalement étroitement le trajet défini par le vaisseau sanguin dans lequel elle est insérée. Cependant, parfois, sa forme peut devenir assez sinueuse après le retrait du stylet, surtout à l'endroit où le vaisseau comporte un embranchement. L'électrode peut alors décrire des courbes serrées simples voire doubles en forme de S, par exemple. Or, si le stylet comporte un simple embout émoussant tel qu'une bille (comme décrit par exemple dans le brevet GB-A-2 064 963) ou encore plus simplement une surface d'extrémité arrondie (comme décrit dans le brevet US-A-2 118 631), il ne se laisse que difficilement insérer dans l'électrode, car dans les courbes, l'embout s'accroche sur les spires jointives de l'électrode tendant à les écarter voire à passer à travers le conducteur et la gaine isolante qui l'entoure. Il y a alors un risque important que le stylet en passant à travers la gaine isolante de l'électrode perce le vaisseau sanguin. Dans ces conditions, les praticiens préfèrent abandonner cette méthode de contrôle à l'aide du stylet et avoir recours à l'examen aux rayons X pour vérifier la bonne implantation.

L'invention a pour but de fournir un stylet qui permette le contrôle de l'implantation sans aucun risque de percement ni du conducteur de l'électrode, ni du vaisseau sanguin.

L'invention a donc pour objet un stylet pour la mise en place et le contrôle d'une électrode implantable du type comprenant un conducteur creux allongé formé d'un enroulement en hélice à spires jointives d'un fil, ledit stylet comprenant un fil élastique qui, lors de l'implantation de l'électrode, est maintenu dans celle-ci pour la rigidifier sur toute sa longueur et qui présente à son extrémité antérieure un embout émoussant à symétrie axiale fixée coaxialement à l'extrémité antérieure du fil, son diamètre étant légèrement inférieur au diamètre intérieur du conducteur de l'électrode, caractérisé en ce que l'embout émoussant présente une forme en ogive.

Grâce à cette forme particulière de l'élément émoussant, l'accrochage sur les spires du conducteur ne peut se produire, même si ce conducteur décrit une courbe serrée, car lorsqu'une poussée axiale dans le sens de l'insertion est appliquée sur le fil élastique, celui-ci par son flambage dû à la résistance apportée par l'élément émoussant, applique un léger couple de basculement sur celui-ci en écartant son extrémité antérieure des spires du conducteur. Ainsi, de proche en proche, l'élément émoussant passe devant les spires jointives dans les courbes du conducteur facilitant ainsi considérablement la progression du stylet.

Le couple de basculement peut être augmenté pour faciliter encore davantage cette progression si, suivant une autre caractéristique de l'invention, l'élément émoussant comporte une cavité cylindrique axiale borgne ouverte vers l'arrière et traversée par l'extrémité antérieure du fil, ce dernier étant fixé à l'embout dans la zone du fond de la cavité, celle-ci ayant un diamètre supérieur à celui du fil élastique.

C'est en effet dans ces conditions que les forces qui créent le couple de basculement peuvent avoir des points d'application aussi éloignés que possible les uns des autres. De la sorte, des courbes encore plus serrées peuvent être franchies sans risque de percement ni de la gaine isolante de l'électrode, ni, à fortiori, de la paroi du vaisseau sanguin.

L'invention sera mieux comprise à l'aide de la description qui va suivre d'un exemple de réalisation.

Aux dessins annexés, donnés uniquement à titre d'exemple:

- la Fig. 1 est une vue à grande échelle et en coupe axiale de la partie antérieure d'un stylet suivant l'invention;
- la Fig. 2 montre un autre mode de réalisation, actuellement préféré;
- la Fig. 3 représente schématiquement comment l'embout du stylet parvient à favoriser la progression du stylet dans une électrode qui est déjà implantée et qui suit un trajet sinueux.

La Fig. 4 montre une vue en coupe à grande échelle de l'embout du stylet suivant la Fig. 3.

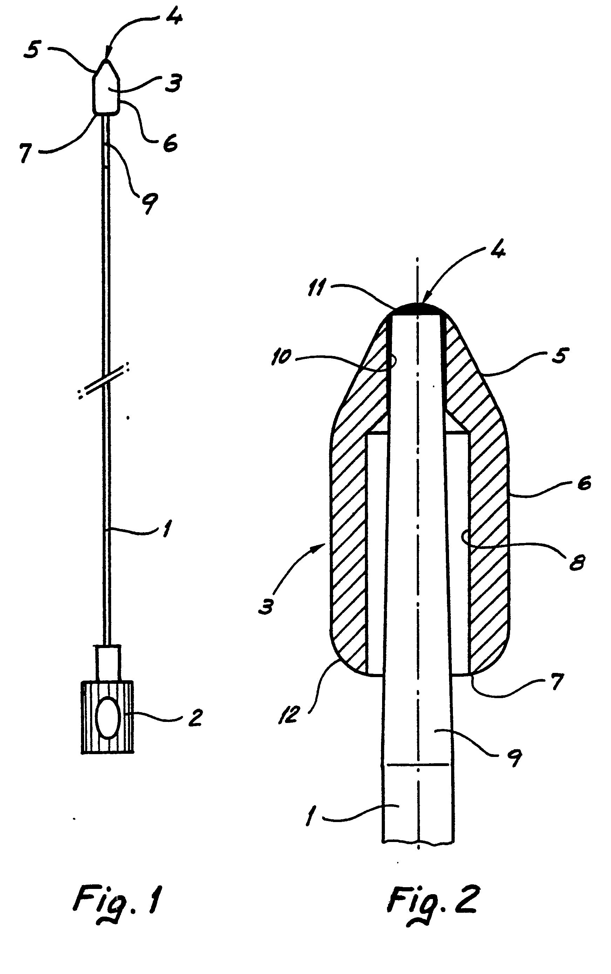

On a montré sur la Fig. 1, une première formé de réalisation d'un stylet suivant l'invention. Ce dernier comporte un fil élastique 1 se terminant à son extrémité postérieure par un bouton 2 destiné à faciliter la préhension par le chirurgien. Dans sa partie antérieure ce fil peut être légèrement tronconique.

Un élément émoussant ou embout 3 est fixé à ce fil. Il présente une forme générale en ogive avec une face d'extrémité 4 arrondie, une surface tronconique 5 qui se raccorde à cette face et à une surface cylindrique 6 qui fait suite à cette dernière. Dans l'ensemble, l'embout 3 présente ainsi une symétrie axiale par rapport à l'axe longitudinal du stylet.

L'assemblage de l'embout et de l'extrémité correspondante du fil est réalisé de préférence par brasage ou par sertissage de cette extrémité dans le corps de l'embout.

L'ensemble du stylet peut être réalisé en acier inoxydable, par exemple.

Par ailleurs et pour fixer les idées, le diamètre le plus grand de l'embout peut être de 0,60 mm pour un diamètre intérieur de 0,80 mm du conducteur (non représenté) d'une électrode implantable à laquelle le stylet est associé. La longueur de l'embout peut être de 1,20 mm, tandis que le diamètre du fil dans sa partie cylindrique peut être de 0,20 mm. Bien entendu, toutes ces dimensions ne sont données qu'à titre indicatif.

Dans la version représentée à la Fig. 1, l'embout 3 est massif, le fil 1 s'étendant librement, seulement à partir de la face postérieure 7 de cet embout.

Par contre, dans le mode de réalisation de la Fig. 2, qui est actuellement préféré, l'embout 3 comporte une cavité axiale borgne 8 de forme générale cylindrique et s'ouvrant sur la face postérieure 7 de l'embout. On remarquera que le diamètre de cette cavité dépasse nettement celui du fil 1; par exemple lorsque celui-ci a un diamètre de 0,20 mm, la cavité peut avoir un diamètre de 0,35 mm.

L'extrémité antérieure du fil, c'est-à-dire sa partie tronconique 9 passe à travers la cavité 8 et est enfilée dans un orifice axial 10 qui prolonge la cavité 8 à l'avant. Afin d'assurer la fixation du fil dans cet orifice 10, il est prévu une soudure 11 à la face d'extrémité 4, cette soudure étant travaillée de telle façon que cette face se raccorde sans discontinuités à la surface tronconique 5. Une autre façon d'assembler l'embout 3 au fil 1 consiste à river ce dernier à la face avant en le faisant légèrement dépasser de l'orifice 10 avant l'opération de rivetage.

On peut noter que grâce à la légère conicité de l'extrémité antérieure 8, on peut positionner facilement le fil dans le sens axial par rapport à l'embout 3 et couper le fil à la longueur souhaitée à l'extrémité avant de l'orifice 10, pour ensuite procéder à l'opération d'assemblage, par soudure ou rivetage.

Il s'est avéré que l'inclinaison de la surface tronconique 5 par rapport à l'axe du stylet doit être soigneusement choisie. Suivant le degré de courbure auquel on peut s'attendre lorsqu'une électrode est implantée, l'angle peut varier entre 15 et 50° environ, mais on a constaté qu'un angle de 26° pouvait assurer une adaptation aux degrés de courbure les plus fréquents de l'électrode implantée.

Il est également avantageux de prévoir un raccord 12 à section courbe entre la paroi latérale de l'embout 3 et sa face postérieure 7 et ceci afin de faciliter le retrait du stylet de l'électrode après l'implantation ou le contrôle de cette dernière.

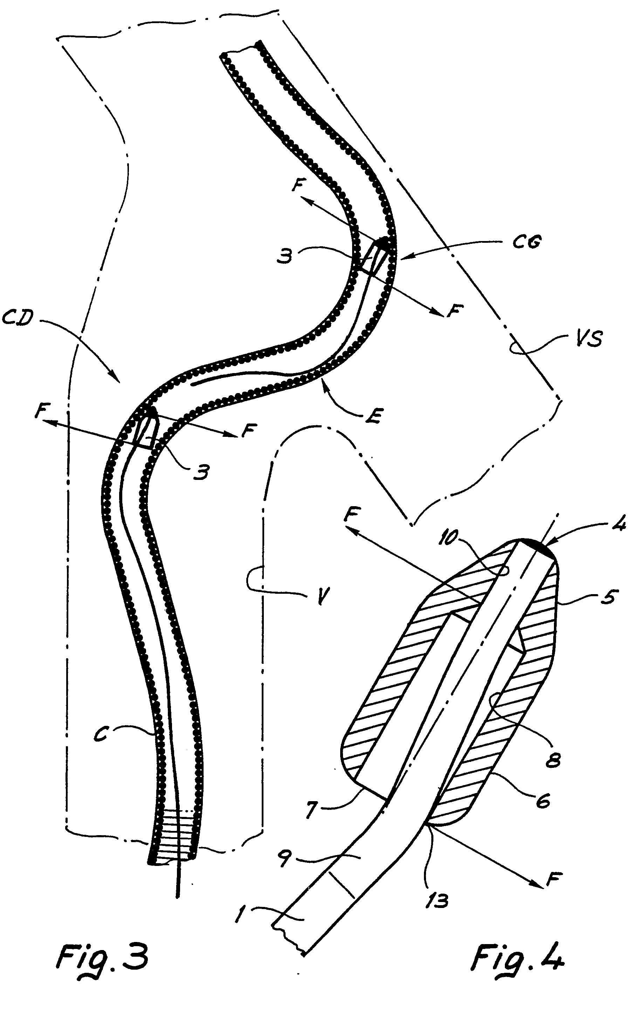

La Fig. 3 illustre le fonctionnement d'un stylet réalisé selon la Fig. 2. Elle montre un vaisseau sanguin V dans lequel passe une électrode E formé d'un enroulement à spires jointives d'un fil C lui-même formé de plusieurs torons enroulés en hélice. On suppose qu'un vaisseau sanguin secondaire VS débouche sur le vaisseau principal V et qu'après quelques jours de présence dans le corps, l'électrode E a formé à cet endroit un coude prononcé avec d'abord une courbe à droite (CD) puis une courbe à gauche (CG) vu de l'extrémité postérieure de l'électrode (en bas sur la Figure).

Lorsque le stylet suivant l'invention n'est soumis à aucune contrainte, il est parfaitement rectiligne, comme représenté à la Fig. 1 et il reprend cette forme chaque fois qu'une contrainte de courbure lui est appliquée. Cependant, il demeure suffisamment souple pour pouvoir s'inscrire dans toutes les courbures qui lui sont imposées par la forme de l'électrode lors de l'implantation ou de l'insertion ultérieure.

Par conséquent, lorsque dans le cas de la Fig. 3, l'embout 3 aborde la courbure à droite, il vient se heurter très rapidement contre la paroi intérieure de l'électrode.

L'effet de blocage est d'autant plus important que cette paroi est formée par des spires contiguës entre lesquelles sont formées des dépressions. C'est ce qui empêchait toute progression des stylets conçus suivant la technique antérieure comportant un simple embout émoussant formé par une bille fine.

Cependant, grâce à la forme particulière de l'embout suivant l'invention, dès qu'une légère contrainte est exercée sur le fil dans le sens axial, contrainte qui est donc due à la force de blocage de l'embout et la force de poussée exercée sur le stylet par le chirurgien, le fil subit un très léger flambage qui a tendance à faire basculer l'embout par rapport à l'axe de la partie antérieure 9 du fil 1. De ce fait, l'embout viendra en contact avec la paroi de l'électrode, non pas par la face 4, mais par la face tronconique 5 qui, en raison de son inclinaison soigneusement choisie, assurera un contact glissant qui favorise la progression du stylet. Ce processus se produit chaque fois que l'embout est arrêté contre la paroi intérieure de l'électrode.

Une analyse très simplifiée fait apparaître que l'embout 3 subit un couple de basculement, F - F, tendant à chaque fois de l'éloigner de la paroi, ou tout au moins d'y appliquer une génératrice de la partie tronconique 5 de l'embout 3.

En raison de la symétrie axiale de celui-ci ce couple de basculement peut être engendré quel que soit le sens de la courbure (c'est-à-dire dans le plan de la Fig. 3 ou hors de ce plan).

Le phénomène qui vient d'être décrit a lieu tant dans le cas de la Fig. 1 que dans celui des Fig. 2 et 4, dont la variante provoque une nette augmentation du couple de basculement du fait que la force d'éloignement est ramenée le plus proche possible vers l'extrémité avant de l'embout 3. Par ailleurs, si le flambage du fil 1 est suffisant, le contact de celui-ci avec la paroi de la cavité en 13 (Fig. 4) renforce encore davantage le phénomène recherché.

## Revendications

1. Stylet pour la mise en place et le contrôle d'une électrode implantable (E) du type comprenant un conducteur creux allongé (F) formé d'un enroulement en hélice à spires jointives d'un fil, ledit stylet comprenant un fil élastique (1) qui, lors de l'implantation de l'électrode (E), est maintenu dans celle-ci pour la rigidifier sur toute sa longueur et qui présente à son extrémité antérieure un embout émoussant (3) à symétrie axiale fixée coaxialement à l'extrémité antérieur (9) du fil (1), son diamètre étant légèrement inférieur au diamètre intérieur du conducteur (F) de l'électrode (E), caractérisé en ce que l'embout émoussant (3) présente une forme en ogive.

2. Stylet suivant la revendication 1, caractérisé en ce que la forme extérieure dudit embout (3) est définie par une surface cylindrique postérieure (6), une face antérieure arrondie (4) et une surface de raccordement tronconique (5) se rétrécissant vers l'avant et se raccordant sans discontinuités à la face avant (4) et à la surface cylindrique (6).

3. Stylet suivant la revendication 2, caractérisé en ce que ladite surface tronconique (5) est inclinée sur l'axe de l'embout d'un angle situé entre 15 et 50°.

4. Stylet suivant la revendication 3, caractérisé en ce que ledit angle est égal à 26°.

5. Stylet suivant l'une quelconque des revendications 1 à 4, caractérisé en ce que ledit embout (3) présente une cavité axiale borgne (8) s'ouvrant vers l'arrière et traversée par l'extrémité antérieure du fil qui est fixé à l'embout dans la zone du fond de ladite cavité, et en ce que le diamètre de cette cavité (8) est nettement supérieur à celui dudit fil (1).

6. Stylet suivant la revendication 5 comportant un fil (1) dont l'extrémité antérieure (9) est de forme tronconique, caractérisé en ce que ledit embout (3) comporte un orifice cylindrique axial (10) de forme cylindrique qui prolonge ladite cavité (8) vers l'avant et en ce que le diamètre dudit orifice correspond à un diamètre de la partie antérieure tronconique du fil.

## Patentansprüche

1. Sonde zum Einsetzen und zur Kontrolle einer einpflanzbaren Elektrode (E) des Typs mit länglichem, aus einer Wendelwicklung eines Drahtes mit aneinanderverlaufenden Wicklungen gebildetem Hohlleiter (F), wobei die genannte Sonde einen elastischen Draht (1) umfasst, der bei Einpflanzung der Elektrode (E) in dieser gehalten wird, um sie auf ihrer gesamten Länge zu versteifen, und der an seinem vorderen Ende eine stumpfe Kappe (3) aufweist, die axial symmetrisch ausgebildet und koaxial am vorderen Ende (9) des Drahtes (1) befestigt ist und deren Durchmesser etwas kleiner ist als der Innendurchmesser des Hohlleiters (F) der Elektrode (E), dadurch gekennzeichnet, dass die stumpfe Kappe (3) eine Ogivenform aufweist.

2. Sonde gemäss Anspruch 1, dadurch gekennzeichnet, dass die äussere Form der genannten Kappe (3) durch eine hintere zylindrische Oberfläche (6), eine abgerundete Vorderfläche (4) und eine sich nach vorn verkleinernde kegelstumpfförmige Zwischenoberfläche (6) definiert ist, wobei letztere sich übergangslos an die Vorderfläche (4) und die zylinderische Oberfläche (6) anschliesst.

3. Sonde gemäss Anspruch 2, dadurch gekennzeichnet, dass die genannte Kegelstumpfoberfläche (5) gegenüber der Kappenachse um einen zwischen 15 und 50° liegenden Winkel geneigt ist.

4. Sonde gemäss Anspruch 3, dadurch gekennzeichnet, dass der genannte Winkel 26° beträgt.

5. Sonde gemäss einem beliebigen der Ansprüche 1 bis 4, dadurch gekennzeichnet, dass die genannte Kappe (3) einen axialen Hohlraum (8) aufweist, der sich nach hinten öffnet und vom vorderen Ende des Drahtes (1) durchquert wird, welcher im hinteren Hohlraumbereich an der Kappe befestigt ist, und dadurch, dass der Durchmesser dieses Hohlraumes (8) beträchtlich grösser ist als der des genannten Drahtes (1).

6. Sonde gemäss Anspruch 5, umfassend einen Draht (1), dessen vorderes Ende (9) kegelstumpfförmig ist, dadurch gekennzeichnet, dass die genannte Kappe (3) ein axial verlaufendes zylindrisches Loch (10) umfasst, das den genannten Hohlraum (8) nach vorn verlängert, und dadurch, dass der Durchmesser des genannten Hohlraums einem Durchmesser des vorderen kegelstumpfförmigen Teils des Drahtes entspricht.

## Claims

1. A stylet for the implantation and for the checking on an implantable electrode (E) of the kind having a hollow elongated conductor (F) of coiled wire forming continuous turns, said stylet comprising an elastic filament (1) which, during implantation of electrode (E), is maintained in it in order to stiffen it over its entire length and which is provided at its front end with a blunting tip (3) having an axially symmetrical shape coaxially secured to the front end (9) of filament (1), and having a diameter slightly smaller than the internal diameter of conductor (F) of electrode (E), characterized in that the blunting tip (3) has a bullet shape.

2. A stylet according to claim 1, characterized in that the outer shape of said tip (3) is defined by a rear cylindrical surface (6), a rounded front face (4) and a frusto-conical transition surface (5) which tapers to the front end and which smoothly merges with the front face (4) and the cylindrical surface (6).

3. A stylet according to claim 2, characterized in that said frusto-conical surface (5) is inclined to the axis of the tip by an angle ranging from 15 to 50°.

4. A stylet according to claim 3, characterized in that said angle is equal to 26°.

5. A stylet according to any one of claims 1 to 4, characterized in that said tip (3) is formed at its rear end with an axial recess (8) into which extends the front end portion of the filament, the latter being secured to the tip in a region proximate to the bottom of said recess, and in that the diameter of this recess (8) is substantially greater than that of the filament (1).

6. A stylet according to claim 5 having a filament the front end portion (9) of which has a frusto-conical shape, characterized in that said tip (3) is formed with an axial, cylindrical aperture (10) extending from said recess (8) from the front end and in that the diameter of said aperture corresponds to a diameter of the frusto-conical front portion of the filament.

Fig. 1

Fig. 2

*Fig.3*

*Fig.4*